# EUROPEAN PATENT APPLICATION

(11) **EP 4 799 999 A1**
(43) Date of publication of application: **02.09.2026**
(21) Application number: 25845566.6
(22) Date of filing: 16.10.2025
(51) Int. Cl.: C07C 23/06, C07C 17/38, C07C 19/08, C11D 7/28

(54) **HIGH-PURITY OCTAFLUOROCYCLOBUTANE**

(30) Priority: 17.10.2024 JP 2024181792
(71) Applicant: DAIKIN INDUSTRIES, LTD., Osaka-Shi, Osaka 530-0001 (JP)
(72) Inventor: NAKAMURA, Shingo, Osaka-shi, Osaka 530-0001 (JP); KOMAYA, Naoki, Osaka-shi, Osaka 530-0001 (JP); YANO, Hiroyuki, Osaka-shi, Osaka 530-0001 (JP); IWAI, Yuki, Osaka-shi, Osaka 530-0001 (JP); INOUE, Reina, Osaka-shi, Osaka 530-0001 (JP); MORIMOTO, Kazunori, Osaka-shi, Osaka 530-0001 (JP); SUGIYAMA, Akinari, Osaka-shi, Osaka 530-0001 (JP)
(74) Representative: Hoffmann Eitle
(86) International application number: PCT/JP2025/036458
(87) International publication number: WO 2026/084016

(57) **Abstract**

An object of the present disclosure is to newly provide high-purity octafluorocyclobutane. High-purity octafluorocyclobutane is provided.

## Description

### Technical Field

The present disclosure relates to high-purity octafluorocyclobutane.

### Background Art

Patent Literature (PTL) 1 discloses a dry etching gas composed of tetrafluoroethylene with a purity of 99.999 wt% or more.

### Citation List

### Patent Literature

PTL 1: WO01/027987

### Summary of Invention

### Technical Problem

An object of the present disclosure is to newly provide high-purity octafluorocyclobutane.

### Solution to Problem

In semiconductor applications, octafluorocyclobutane (c-C₄F₈) with 5N purity (99.999%) has been conventionally used. The influence of impurities cannot be ignored for c-C₄F₈ with 5N purity in semiconductor applications, storage, etc.

The present disclosure includes the following high-purity octafluorocyclobutane.

### Item 1.

A composition comprising (1) octafluorocyclobutane, the content of the octafluorocyclobutane (1) being 99.99999 vol% or more based on the total amount of the composition.

### Item 2.

The composition according to Item 1, further comprising (2) an additional compound, the content of the additional compound (2) being 0.0001 vol% (1 ppm by volume) or less based on the total amount of the composition.

### Item 3.

The composition according to Item 1, wherein the additional compound (2) is at least one component selected from the group consisting of noble gases, inert gases, NH₃, H₂, hydrocarbons, O₂, oxygen compounds, iodine compounds, hydrofluorocarbons (HFCs), and perfluorocarbons (PFCs).

### Item 4.

The composition according to Item 1, wherein the additional compound (2) is at least one component selected from the group consisting of
noble gases selected from the group consisting of He, Ne, Ar, Xe, and Kr;
hydrocarbons selected from the group consisting of CH₄, C₂H₆, C₃H₈, C₂H₄, and C₃H₆;
oxygen compounds selected from the group consisting of CO, CO₂, (CF₃)₂C=O, CF₃CFOCF₂, and CF₃OCF₃ ;
iodine compounds selected from the group consisting of CF₃I, CF₃CF₂I, (CF₃)₂CFI, and CF₂=CFI;
HFCs selected from the group consisting of CH₂F₂, CH₃F, CHF₃, CF₃CHF₂, CHF₂CHF₂, CF₃CH₂F, CHF₂CH₂F, CF₃CH₃, CH₂FCH₂F, CF₂=CHF, CHF=CHF, CH₂=CF₂, CH₂=CHF, CF₃CH=CF₂, CF₃CH=CH₂, and CH₃CF=CH₂;
PFCs selected from the group consisting of CF₄, C₂F₆, C₃F₈, CF₂=CFCF₂CF₃, CF₃CF=CFCF₃, C₄F₁₀, CF₂=CF₂, CF₂=CFCF=CF₂, CF₃CF=CFCF=CF₂, and c-C₅F₈;
N₂ (inert gas);
NH₃;
H₂;
O₂;
H₂O;
HF; and
metal components.

### Item 5.

The composition according to Item 1, wherein the additional compound (2) is at least one component selected from the group consisting of N₂, O₂, H₂, CH₄, Ar, H₂O, HF, metal components, CF₂=CFCF₂CF₃, CF₃CF=CFCF₃, and C₄F₁₀.

### Item 6.

The composition according to any one of Items 1 to 5, wherein the composition is a cleaning agent for use in the production of integrated circuits, or a polymerization solvent.

### Item 7.

A method for storing a composition comprising (1) octafluorocyclobutane, the method comprising adjusting the content of the octafluorocyclobutane (1) to 99.99999 vol% or more based on the total amount of the composition.

### Item 8.

A method for suppressing a decrease in the purity of octafluorocyclobutane in a composition comprising (1) octafluorocyclobutane, the method comprising adjusting the content of the octafluorocyclobutane (1) to 99.99999 vol% or more based on the total amount of the composition.

The composition containing high-purity octafluorocyclobutane (c-C₄F₈) of the present disclosure is a high-purity 7N (99.99999 vol% or more) rectified product, has good storage stability, can be stored in a state in which its physical properties are stable, and can then be used satisfactorily. The present disclosure makes it possible to provide high-purity (7N) c-C₄F₈ for various applications such as solvents (cleaning agents) and polymerization solvents in the field of semiconductor integrated circuits.

### Advantageous Effects of Invention

The present disclosure newly provides high-purity octafluorocyclobutane.

### Description of Embodiments

In the present specification, the terms "comprise," "contain," and "include" encompass the concepts of comprising, consisting essentially of, and consisting of. In the present specification, a numerical range indicated as "A to B" means A or more and B or less. In the present specification, when expressions such as "part(s)" and "%" are used, these mean part(s) by mass, part(s) by weight, mass%, or wt%.

### [1] Composition Containing Octafluorocyclobutane (c-C₄F₈)

Rapid developments in integrated circuit (IC) technology have led to advances in miniaturization and integration, as seen in semiconductor devices, such as ultralarge scale integration (ULSI); thus, chip integration densities have increased, and demands on the performance and precision of transistor devices are increasing. In integrated circuit production, as chip production technology advances to the nanometer scale, impurities in the materials used directly affect the uniformity and stability of chip production technology; thus, there is a need to raise the purity requirements of the materials used.

Octafluorocyclobutane (c-C₄F₈) is a stable chemical substance with low greenhouse gas potential (low global warming potential) and is commonly used in the semiconductor industry. c-C₄F₈ has a relatively low ratio of carbon to fluorine, and also serves as a protective gas in integrated circuit production. In integrated circuit production, impurities in the materials used lead to process variations, resulting in decreased process uniformity. Accordingly, in integrated circuit production, the use of higher-purity c-C₄F₈ is desired to achieve high-precision processes.

### (1) Octafluorocyclobutane (c-C₄F₈)

The composition containing c-C₄F₈ of the present disclosure contains (1) octafluorocyclobutane (perfluorocyclobutane, c-C₄F₈) as a first component.

The composition containing c-C₄F₈ contains the c-C₄F₈ (1), which is the first component, as a main component. In the composition containing c-C₄F₈, the content of the c-C₄F₈ (1) based on the total amount (gas volume) of the composition is 99.99999 vol% or more, which is high-purity 7N. The influence of impurities, even in trace amounts, in the composition containing c-C₄F₈ on c-C₄F₈ is unpredictable. For example, it is preferable to adjust the amounts of substances such as difluoromethane, trifluoroethylene, hydrogen, and carbon monoxide to be equal to or less than predetermined levels to minimize their effects on devices.

In the composition containing c-C₄F₈, the content of the c-C₄F₈ (1) based on the total amount of the composition is 99.99999 vol% or more, and preferably in the following order: 99.999995 vol% or more, 99.999999 vol% or more, 99.9999995 vol% or more, and 99.9999999 vol% or more.

To separate impurities from the composition containing c-C₄F₈, a rectification operation is preferably used as a separation method. To obtain a composition with a c-C₄F₈ content of 99.99999 vol% or more, it is preferable to separate c-C₄F₈ using a rectification column with a large number of plates, and in particular, separation using a rectification column with a very large number of plates is preferable.

In the composition containing c-C₄F₈, the concentration of c-C₄F₈ is adjusted within the above range, which makes it possible to suppress the decomposition of c-C₄F₈ and suppress a decrease in the purity of c-C₄F₈ in the composition containing c-C₄F₈ during transportation of the composition containing c-C₄F₈ in cylinders or the like, whereby storage stability of the composition containing c-C₄F₈ can be well maintained. The composition containing c-C₄F₈ can be stably transported by suppressing the decomposition of c-C₄F₈ even under harsh maritime transportation conditions in containers or the like (e.g., conditions such as temperature increase during transportation).

### (2) Additional Compound

The composition containing c-C₄F₈ of the present disclosure preferably further contains (2) an additional compound as a second component. In the composition containing c-C₄F₈, the content of the additional compound (2) is preferably 0.0001 vol% (1 ppm by volume, ppmv (volume)) or less based on the total amount (gas volume) of the composition. The content of the additional compound, when two or more additional compounds are mixed (blended), refers to the total content of the additional compounds. The influence of impurities, even in trace amounts, in the composition containing C-C₄F₈ on c-C₄F₈ is unpredictable. For example, it is preferable to adjust the amounts of substances such as CH₂F₂, CHF=CF₂, H₂, and CO to be equal to or less than predetermined levels to minimize their effects on devices.

The additional compound is preferably at least one component selected from the group consisting of noble gases, inert gases, NH₃, H₂, hydrocarbons, O₂, oxygen compounds, iodine compounds, hydrofluorocarbons (HFCs), and perfluorocarbons (PFCs).

The additional compound is preferably a noble gas (one or more noble gases) selected from the group consisting of He, Ne, Ar, Xe, and Kr. Noble gases have a gas dilution effect.

The additional compound is preferably a hydrocarbon (one or more hydrocarbons) selected from the group consisting of CH₄, C₂H₆, C₃H₈, C₂H₄, and C₃H₆.

The additional compound is preferably an oxygen compound (one or more oxygen compounds) selected from the group consisting of CO, CO₂, (CF₃)₂C=O, CF₃CFOCF₂, and CF₃OCF₃.

The additional compound is preferably an iodine compound (one or more iodine compounds) selected from the group consisting of CF₃I, CF₃CF₂I, (CF₃)₂CFI, and CF₂=CFI.

The additional compound is preferably an HFC (one or more HFCs) selected from the group consisting of CH₂F₂, CH₃F, CHF₃, CF₃CHF₂, CHF₂CHF₂, CF₃CH₂F, CHF₂CH₂F, CF₃CH₃, CH₂FCH₂F, CF₂=CHF, CHF=CHF, CH₂=CF₂, CH₂=CHF, CF₃CH=CF₂, CF₃CH=CH₂, and CH₃CF=CH₂. HFCs can control the balance between fluorocarbon radicals and fluorocarbon ions.

The additional compound is preferably a PFC (one or more PFCs) selected from the group consisting of CF₄, C₂F₆, C₃F₈, CF₂=CFCF₂CF₃, CF₃CF=CFCF₃, C₄F₁₀, CF₂=CF₂, CF₂=CFCF=CF₂, CF₃CF=CFCF=CF₂, and c-C₅F₈.

The additional compound is preferably a component (one or more components) selected from the group consisting of N₂ (inert gas); NH₃, H₂, O₂; H₂O; HF; and metal components.

The composition containing c-C₄F₈ may contain a single additional compound or may contain a mixture (blend) of two or more additional compounds.

The additional compound is preferably at least one component selected from the group consisting of N₂, O₂, H₂, CH₄, Ar, H₂O, HF, metal components, CF₂=CFCF₂CF₃, CF₃CF=CFCF₃, and C₄F₁₀.

In the composition containing c-C₄F₈, the content of the additional compound (2) based on the total amount of composition is preferably 0.0001 vol% (1 ppm by volume) or less, and more preferably in the following order: 0.00005 vol% (0.5 ppm by volume) or less, 0.00001 vol% (0.1 ppm by volume) or less, 0.000005 vol% (0.05 ppm by volume) or less, and 0.000001 vol% (0.01 ppm by volume) or less.

Problematic additional compounds (or impurities) in the composition containing c-C₄F₈ are hydrogen-containing compounds (e.g., CH₂F₂ and CHF=CF₂), H₂, CO, etc. By adjusting the total amount of these additional compounds to 0.0001 vol% (1 ppm by volume) or less, favorable results can be consistently achieved in the production of highly miniaturized semiconductor devices.

The composition containing c-C₄F₈ of the present disclosure has a c-C₄F₈ content of 99.99999 vol% or more, which is high-purity 7N. The use of the composition containing c-C₄F₈ of the present disclosure enables highly miniaturized and highly integrated semiconductor devices to be stably produced with less adverse effects from impurities.

In the composition containing c-C₄F₈, the concentration of the additional compound is adjusted within the above range, which makes it possible to suppress the decomposition of c-C₄F₈ and suppress a decrease in the purity of c-C₄F₈ in the composition containing c-C₄F₈ during transportation of the composition containing c-C₄F₈ in cylinders or the like, whereby storage stability of the composition containing c-C₄F₈ can be well maintained. The composition containing c-C₄F₈ can be stably transported by suppressing the decomposition of c-C₄F₈ even under harsh maritime transportation conditions in containers or the like (e.g., conditions such as temperature increase during transportation).

### [2] Method for Storing Composition Containing Octafluorocyclobutane (c-C₄F₈)

The present disclosure includes a method for storing a composition containing c-C₄F₈.

The present disclosure includes a method for suppressing a decrease in the purity of c-C₄F₈ in a composition containing c-C₄F₈.

The description in section "[1] Composition Containing c-C₄F₈" above is applied to the description of the composition containing c-C₄F₈.

The present disclosure discloses a method for stably transporting a composition containing c-C₄F₈. According to the present disclosure, the concentration of c-C₄F₈ in the composition containing c-C₄F₈ is adjusted, which makes it possible to suppress the decomposition of c-C₄F₈ and suppress a decrease in the purity of c-C₄F₈ in the composition containing c-C₄F₈ during transportation of the composition containing c-C₄F₈ in cylinders or the like, whereby storage stability of the composition containing c-C₄F₈ can be well maintained.

The composition containing c-C₄F₈ of the present disclosure contains c-C₄F₈ and suppresses the generation of a c-C₄F₈-derived decomposed product. Thus, the composition has improved storage stability and can be stably transported by suppressing the decomposition of c-C₄F₈ even under harsh maritime transportation conditions in containers or the like (e.g., conditions such as temperature increase during transportation).

### [3] Method for Suppressing Decrease in Purity of c-C₄F₈ in Composition Containing Octafluorocyclobutane (c-C₄F₈)

The composition containing c-C₄F₈ of the present disclosure improves process stability and can be applied to advanced processes in integrated circuit production, which leads to improved device uniformity and yield.

The composition containing c-C₄F₈ can be suitably applied to a cleaning agent for use in integrated circuit production, or a polymerization solvent.

The present disclosure discloses a method for stably transporting a composition containing c-C₄F₈. According to the present disclosure, since the composition containing c-C₄F₈ contains high-purity 7N (99.99999 vol% or more) rectified c-C₄F₈, the decomposition of c-C₄F₈ and a decrease in the purity of c-C₄F₈ are suppressed during transportation of the composition in cylinders or the like, whereby the storage stability of the composition containing c-C₄F₈ can be maintained.

The composition containing c-C₄F₈ of the present disclosure contain high-purity 7N rectified c-C₄F₈, has improved storage stability, and can be stably transported by suppressing the decomposition of c-C₄F₈ even under harsh maritime transportation conditions in containers or the like (e.g., conditions such as temperature increase during transportation).

### Examples

The present disclosure invention is specifically described below with reference to Examples and Comparative Examples, which do not limit the present disclosure.

### [1] Storage Stability of Composition Containing Octafluorocyclobutane (c-C₄F₈)

Octafluorocyclobutane: c-C₄F₈
Additional compound: C₄F₁₀

A composition containing c-C₄F₈ was prepared by purifying c-C₄F₈ (high-purity 7N, 99.99999 vol% or more) and adjusting the concentration of the additional compound, i.e., C₄F₁₀ to a predetermined level (0.0001 mass% (1 ppm by volume) or less).

The concentration (ppm by volume) of c-C₄F₈ and the concentration (ppm by volume) of the additional compound, i.e., C₄F₁₀ in the composition containing c-C₄F₈ were analyzed by gas chromatography (GC analysis).

The composition containing c-C₄F₈ was stored at a constant temperature (20°C or 50°C). After fixed periods of time (30 days, 90 days, and 180 days), the gas phase was extracted, and a c-C₄F₈-derived decomposed product was analyzed by GC analysis (ppm by volume).

The composition containing c-C₄F₈ of the Example contained high-purity 7N (99.99999 vol% or more) c-C₄F₈, and the amount of the additional compound, i.e., C₄F₁₀ in the composition was adjusted to 0.0001 mass% (1 ppm by volume) or less; thus, the composition enabled the generation of a c-C₄F₈-derived decomposed product to be satisfactorily suppressed even after the fixed periods of time (30 days, 90 days, and 180 days) of storage at a constant temperature (20°C or 50°C).

### [2] Industrial Applicability

The composition containing c-C₄F₈ of the present disclosure improves process stability and can be applied to advanced processes in integrated circuit production, which leads to improved device uniformity and yield.

## Claims

1. A composition comprising (1) octafluorocyclobutane, the content of the octafluorocyclobutane (1) being 99.99999 vol% or more based on the total amount of the composition.

2. The composition according to claim 1, further comprising (2) an additional compound, the content of the additional compound (2) being 0.0001 vol% (1 ppm by volume) or less based on the total amount of the composition.

3. The composition according to claim 1, wherein the additional compound (2) is at least one component selected from the group consisting of noble gases, inert gases, NH₃, H₂, hydrocarbons, O₂, oxygen compounds, iodine compounds, hydrofluorocarbons (HFCs), and perfluorocarbons (PFCs).

4. The composition according to claim 1, wherein the additional compound (2) is at least one component selected from the group consisting of
noble gases selected from the group consisting of He, Ne, Ar, Xe, and Kr;
hydrocarbons selected from the group consisting of CH₄, C₂H₆, C₃H₈, C₂H₄, and C₃H₆;
oxygen compounds selected from the group consisting of CO, CO₂, (CF₃)₂C=O, CF₃CFOCF₂, and CF₃OCF₃;
iodine compounds selected from the group consisting of CF₃I, CF₃CF₂I, (CF₃)₂CFI, and CF₂=CFI;
HFCs selected from the group consisting of CH₂F₂, CH₃F, CHF₃, CF₃CHF₂, CHF₂CHF₂, CF₃CH₂F, CHF₂CH₂F, CF₃CH₃, CH₂FCH₂F, CF₂=CHF, CHF=CHF, CH₂=CF₂, CH₂=CHF, CF₃CH=CF₂, CF₃CH=CH₂, and CH₃CF=CH₂;
PFCs selected from the group consisting of CF₄, C₂F₆, C₃F₈, CF₂=CFCF₂CF₃, CF₃CF=CFCF₃, C₄F₁₀, CF₂=CF₂, CF₂=CFCF=CF₂, CF₃CF=CFCF=CF₂, and c-C₅F₈;
N₂;
NH₃;
H₂;
O₂;
H₂O;
HF; and
metal components.

5. The composition according to claim 1, wherein the additional compound (2) is at least one component selected from the group consisting of N₂, O₂, H₂, CH₄, Ar, H₂O, HF, metal components, CF₂=CFCF₂CF₃, CF₃CF=CFCF₃, and C₄F₁₀.

6. The composition according to any one of claims 1 to 5, wherein the composition is a cleaning agent for use in the production of integrated circuits, or a polymerization solvent.

7. A method for storing a composition comprising (1) octafluorocyclobutane, the method comprising adjusting the content of the octafluorocyclobutane (1) to 99.99999 vol% or more based on the total amount of the composition.

8. A method for suppressing a decrease in the purity of octafluorocyclobutane in a composition comprising (1) octafluorocyclobutane, the method comprising adjusting the content of the octafluorocyclobutane (1) to 99.99999 vol% or more based on the total amount of the composition.
